# EUROPEAN PATENT APPLICATION

(11) **EP 1 729 132 A1**
(43) Date of publication of application: **06.12.2006**
(21) Application number: 05727132.2
(22) Date of filing: 24.03.2005
(51) Int. Cl.: G01N 33/68, C12M 1/40, C12Q 1/26, G01N 3/00, G01N 33/15, G01N 33/50, G01N 33/543

(54) **METHOD OF DETECTING AMYLOID STRUCTURAL CHANGE IN PROTEIN, METHOD OF SEARCHING FOR SUBSTANCE HAVING ACTIVITY OF AFFECTING AMYLOID STRUCTURAL CHANGE AND METHOD OF SEARCHING FOR REMEDY OR DIAGNOSTIC FOR AMYLOID-RELATED DISEASE**

(30) Priority: 25.03.2004 JP 2004089282
(71) Applicant: Fuence Co., Ltd., Tokyo 150-0012 (JP)
(72) Inventor: TACHIBANA, Masayoshi, Niigata-shi, Niigata 951-8104 (JP); NONAKA, Hiromi, FUENCE CO., LTD., Tokyo 150-0012 (JP); KASE, Hiroshi, FUENCE CO., LTD., Tokyo 150-0012 (JP)
(74) Representative: Knuth-Lehtola, Sisko Hillevi
(86) International application number: PCT/JP2005/005410
(87) International publication number: WO 2005/093430

(57) **Abstract**

The inventors investigated to provide a means for real-time detection and measurement on the process of the amyloid protein aggregation as change(s) in tension and/or elasticity using a force sensor. The present invention provided a method of detecting an amyloidgenic conformational change of a protein using a mechanochemical sensor. The present invention further provided a method of searching a substance having an activity that affects to an amyloidgenic conformational change using said detecting method, as well as a method of searching a therapeutic or diagnostic agent for amyloid-related diseases. The method of the present invention is assumed to contribute to obtain a novel therapeutic or diagnostic agent for amyloid diseases.

## Description

### BACKGROUND OF THE INVENTION

### 1. FIELD OF THE INVENTION

The present invention relates to a method of detecting conformational change of an amyloid protein using a mechanochemical sensor. The present invention further relates to a method of searching a substance having an activity that affects to an amyloidgenic conformational change by detecting conformational change of an amyloid protein using a mechanochemical sensor, as well as a method of searching a therapeutic or diagnostic agent for amyloid-related diseases.

### 2. RELATED ART

Amyloid-related disorders including Alzheimer's disease, immunocytic amyloidosis, reactive amyloidosis and familial amyloidosis are caused by protein misfolding. There are medical agents that can restrain progression of Alzheimer disease temporarily, however, such amyloid-related disorders are intractable in many cases. In human, about twenty proteins have been known to form amyloid fibril misfolding, such proteins do not share homology with each other.

The amyloid having such structure is a kind of fibrous protein, and amyloid fibrils are deposited in the blood vessel or other tissues in a body under a certain pathological condition to cause a functional disorder. The amyloid fiber comprises a β-sheet structure, composed of several thousands proteins or peptides bound together by non-covalent bonding. It has been suggested that the diseases may be restrained, by inhibiting the amyloidgenic conformational change and increasing instability of the amyloid protein ( P. Hammarstrom et al., Science, 2001, 293, 2459). As stated above, it is of great importance to detect the conformational change of the amyloid protein, and a therapeutic agent for amyloid-related diseases may be found by searching a chemical compound that inhibits the amyloidgenic conformational change.

The evaluation on the activity to form aggregation of an amyloid protein and screening of compounds have been carried out in various methods such as measurement of turbidity (J. Jarrett et al., Biochemistry, 1993, 32, 4693), determination of the thioflavine T binding site (H. Naiki et al., Lab. Invest., 1991, 65, 104, H. LeVine, Protein Sci., 1993, 2, 404), congo red dyeing (E. M. Castano et al., Biochem. Biophys. Res. Commun., 1986, 141, 782), fluoroscopic method (T. H. J. Huang et al., J. Mol. Biol., 1997, 269, 214), measurement of scattered light using raman spectrum (T. Miura et al., Biochemistry, 2000, 39, 7024) and a method of observational using NMR or an electronic microscope (X. Wu et al., the 224th ACS National meeting and Exposition, 2002, C. Soto et al., J. Biol. Chem., 1995, 270, 3063).

In these methods, however, it generally takes several hours to a week to prepare a protein aggregation to be detected, thus it takes too long time to achieve the evaluation. Therefore, by using a sensor that can detect aggregation of an amyloid protein in a short period of time, the activity can be evaluated effectively.

Further, since these methods use a technique of measuring and observing the result of aggregation, there has been no means for measuring the process of aggregation in real time using the alteration(s) in tension and/or elasticity as indicator(s), by a force sensor or the like.

### SUMMARY OF THE INVENTION

The present invention therefore is intended to provide a means for detecting and measuring the process of aggregation of an amyloid protein in real time as change(s) in tension and/or elasticity using a force sensor. In this way, the conformational change associated with aggregation of the amyloid protein can be detected easily and simply in a short period of time.

The inventors of the present invention completed this invention by finding the knowledge that the amyloidgenic conformational change that arises when a test sample is added to a sample film comprising the amyloid protein can be detected based on change(s) in tension and/or elasticity of the sample film. The method of the present invention is considered to be effective in searching a substance having an activity to affect to the conformational change of the amyloid protein, among various kinds of substances.

The present invention therefore is intended to provide a method of detecting an amyloidgenic conformational change of a protein, the method comprises; forming a sample film on a substrate, the sample film comprising a protein that arises an amyloidgenic conformational change, a fragment of said protein, a variant of said protein, said protein added with a tag, or an antibody protein against said protein, placing said substrate comprising said sample film to a force sensor, and detecting change(s) in tension and/or elasticity of said sample film when a test sample is subjected to said sample film by said force sensor.

Further, the present invention is intended to provide a method of searching a substance having an activity that affects to an amyloidgenic conformational change, the method comprises; forming a sample film on a substrate, the sample film comprising a protein that arises an amyloidgenic conformational change, a fragment of said protein, a variant of said protein, said protein added with a tag, or an antibody protein against said protein, placing said substrate comprising said sample film to a force sensor, and detecting change(s) in tension and/or elasticity of said sample film when a test sample is subjected to said sample film by said force sensor.

Furthermore, the present invention is intended to provide a method of searching a therapeutic or diagnostic agent for amyloid-related diseases, the method comprises: forming a sample film on a substrate, the sample film comprising a protein that arises an amyloidgenic conformational change, a fragment of said protein, a variant of said protein, said protein added with a tag, or an antibody protein against said protein, placing said substrate comprising said sample film to a force sensor, and detecting change(s) in tension and/or elasticity of said sample film when a test sample is subjected to said sample film by said force sensor.

This invention provided a method of detecting an amyloidgenic conformational change of a protein using a mechanochemical sensor, a method of screening a therapeutic or diagnostic agent for amyloid-related diseases using said method, as well as a method of searching a substance having an activity that affects to an amyloidgenic conformational change. The method of the present invention is assumed to contribute to obtain a novel therapeutic or diagnostic agent for amyloid diseases.

### BRIEF DESCRIPTION OF THE DRAWINGS

[FIG.1] FIG.1 is a graph showing the effect of ZnCl₂ and EDTA on the changes in tension and elasticity of amyloid β (1-42).
[FIG.2] FIG.2 is a graph showing dose-dependency of ZnCl₂ on the changes in tension and elasticity of amyloid β(1-42).
[FIG.3] FIG.3 is a graph showing the effect of CuCl₂ and EDTA on the changes in tension and elasticity of amyloid β (1-42).
[FIG.4] FIG.4 is a graph showing dose-dependency of ZnCl₂ on the changes in tension and elasticity of α-synuclein.

### BEST MODE FOR CARRYYING OUT THE INVENTION

Conventionally, the activity to form aggregation of amyloid proteins has been detected by producing aggregation of an amyloid protein, however, it takes a lot of time and energy. According to the present invention, using a force sensor, detection of conformational change of an amyloid protein was enabled easily and simply at real time.

Herein, "a protein that arises an amyloidgenic conformational change" means a protein that arises a conformational change to form an amyloid fibril comprising a characteristic and layered β-sheet structure, and such protein causes amyloid-related diseases. The proteins known to arise the amyloidgenic conformational change may include, but not limited to, amyloid β protein, immunoglobulin light chain protein, amyloid A protein, transthyretin protein, lysozyme, BriL protein, cystatin C protein, scrapie protein, β2 microglobulin, apolipoprotein A1, gelsolin, pancreatic islet amyloid protein, fibrinogen, prolactin, insulin, calcitonin, atrial natriuretic peptide, α-synuclein, prion protein, huntingtin protein, superoxide dismutase, α1-antichymotrypsin, and tau protein. Among all these, amyloid β protein is well-known as a typical protein that arises amyloidgenic conformational change.

The most prominent feature of present invention lies in that the amyloidgenic conformational change of such protein can be detected based on the change in the mechanical characteristics of a sample film comprising said protein. According to the possible embodiment this invention, the changes in the mechanical characteristics of the sample film can be measured using either one of tension or elasticity, or both of tension and elasticity as an index, however, the present invention is not limited to these specific embodiments described above.

In the present invention, at first, a sample film comprising a protein that arises an amyloidgenic conformational change may be prepared on a substrate. The size of the sample film may preferably be 50 to 1000 µm in length, 200 to 2000 µm in width, and 0.3 to 10 µm in thickness, but not limited to these dimensions. Also, the substrate in the present invention may be an appropriate film support, which enables to carry the sample film to a measuring apparatus, by preparing the sample film on the support. The material and size of the substrate is not particularly limited.

Moreover, not only a protein that arises an amyloidgenic conformational change, a fragment and a variant protein of said protein can be also used, as long as retaining the activity to arise an amyloidgenic conformational change. Also, said protein may be attached with a tag for the convenience of detection. Furthermore, the film may be prepared from an antibody protein against said protein, and said film comprising the antibody protein may be bound with said protein may be also used to obtain similar effects.

The methods of preparing such protein film may include an ESD method which comprises forming a thin layer by depositing the sample using electrospray, and a drying method which comprises forming a film by drying a solution. Those techniques are known among those skilled in the art, so they can use such techniques for the purpose of this invention with proper modification. As an example of such reference disclosing such a technique, WO 2002-511792 can be listed, which describes a method of producing a deposition of nonvolatile substances including macro-biomolecules using electrospray.

Moreover, Japanese Patent Publication No.2003-136005 describes a device for immobilizing macro-biomolecules and the like to form thin layers and spots while retaining the activities of the biomolecules. Furthermore, WO 2002-503332 describes a method and an apparatus for measuring binding of a ligand to a DNA or a protein. According to the apparatus described in WO 2002-503332, the effect of chemicals to a sample film comprising biomacromolecules can be measured mechanochemically. Therefore, changes in tension and/or elasticity of the sample film can be detected using the apparatus described in WO 2002-503332, as a preferred embodiment of the present invention.

Now, mechanochemical methods for measuring the elasticity of a protein film, to measure the interaction between a ligand and a protein, are described in V.N, Morozov and T.Ya. Morozova (1992) Anal. Biochem., 201:68-79 and in V.N, Morozov and T.Ya. Morozova (1984) FEBS Letters, 175:299-302. Those skilled in the art can achieve appropriate modifications with reference to these documents to carry out the present invention.

Moreover, an intermediate layer comprising water-soluble polymer can be provided between said substrate and said sample film, as a preferred embodiment of the present invention. In the following examples, 1.2% polyvinyl pyrrolidone (PVP) is used as such an intermediate layer. Such an intermediate layer facilitates detachment of the sample film from the substrate. When PVP is used as the intermediate layer, concentration of the PVP may be 0.1 to 5%, preferably 0.3 to 2 %, but the concentration of PVP is not particularly limited. Other water-soluble polymers may be also used.

As described in WO 2002-503332, examples of materials which can be used as this intermediate layer may include: (1) a water-soluble polymer layer, such as polyacrylamide or polyethylene glycol; (2) a layer of polymer having disulfide bonds which can be reduced by mercaptoethanol; (3) a layer of highly dispersed carbon having low adherence to the deposited biomolecules; and (4) a layer of conductive composites of carbonpolymers having low melting point.

Said protein can be then immobilized by cross-linking the protein comprising said sample film. Such cross-linking is effective for the purpose to maintain the form and strength of the sample film itself. Cross-linking reagents available for polymerizing biomolecules are well-known to those skilled in the art. For instance, Hermanson et al., Immobilized Affinity Ligand Techniques Academic Press, New York, 1991 can be used as a reference.

As a reagent used for cross-linking protein, glutaraldehyde, used in the following examples, is the most preferred. Moreover, the reagents for protein cross-linking may include, but are not limited to, zero-length cross-linking reagents such as 1-ethyl-3-(3-dimethylamino) propyl carbodiimide (EDC); homo-bifunctional cross-linking reagents such as dimethyl adipinimidate (DMA); hetero-bifunctional cross linking reagents such as succinimidyl 3-(2-pyridyldithio)propionate (SPDP); and trifunctional cross-linking reagents such as 4-azide-2-nitrophenylbiocytin-4-nitrophenyl ester. Further, the time period for cross-linking reaction is not specifically limited. The time period for cross-linking glutaraldehyde used in the following examples is five minutes, but the optimum condition may be selected accordingly within the range of about 0 to 3 hours.

The sample film thus prepared can be placed to the detecting apparatus described in WO 2002-503332, then immersed into an appropriate buffer solution to prepare for subjecting the a test sample to the sample film. The buffer solution to be used here may include, but not limited to, Hepes buffer and Tris buffer commonly used in this art. The pH of the buffer solution is not particularly limited either. The appropriate pH may be selected accordingly within the range of about pH3 to pH9.

Furthermore, said buffer solution may have an appropriate salt intensity. It is a preferred embodiment of the present invention to add about 0.15 M of sodium chloride to the buffer solution, as described in the following EXAMPLES. Measurement can be conducted without adding an electrolyte for giving the salt intensity, and such embodiment is also within the scope of the present invention. Further, the electrolyte to be added is not limited to sodium chloride.

After flowing said buffer solution at a constant flow rate to stabilize the tension of the sample film, said buffer solution may be replaced by a buffer solution containing a test sample which is the target of the assay, and it may subjected to the sample film. The change(s) in tension and/or elasticity of the sample film may be measured before and after addition of the test sample using a force sensor and their effects on the conformational change of amyloid protein may be evaluated. The change(s) in tension and/or elasticity can be measured by a force sensor, preferably by a mechanochemical sensor. The measurement may be conducted by a mechanochemical sensor using an apparatus described in WO 2002-503332, as a particularly preferred embodiment of the present invention.

Furthermore, various substances can be used as the test sample to be examined on their effects to the amyloidgenic conformational change of a protein. The substances which can be used as the test sample may include, but are not limited to, protein, peptide, amino acid, sugar, lipid, nucleic acid, metal and organic compound.

The present invention enables to detect alteration(s) in tension and/or elasticity of an amyloid protein rapidly at real time. Therefore, the amyloidgenic conformational change can be evaluated effectively in many samples, so the time needed for searching substances inhibiting the amyloidgenic conformational change can be extremely shortened and substances having such activity can be easily selected from massive substances. Furthermore, a substance that inhibits the amyloidgenic conformational change can be a good candidate for a therapeutic or diagnostic agent for amyloidal diseases. In concrete, it is assumed that if a substance that inhibits the amyloidgenic conformational change can be obtained, further investigation on safety or the like can be conducted, thus a novel therapeutic agent for amyloid diseases can be developed.

### EXAMPLES

The present invention will be described in detail below with reference to examples and figures, but the scope of the present inventions is not to be limited by the descriptions.

### EXAMPLE 1: Effect of ZnCl₂ on the changes in tension and elasticity of amyloid β

Amyloid β (1-42) (Bachem AG, Budendorf, Switzerland) was dissolved in 0.1% ammonia water at the concentration of 1mg/mL. This solution was sprayed under dry air using an electrospry device described in WO 2002-511792 or an immobilzing device described in Japanese Patent Publication No.2003-136005. The solution was permeated through a mask with holes of 400 µm in length and 800 µm in width, and then a film having 1 µm thickness was prepared on 1.2% polyvinyl pyrrolidone, using an electrospray method (the EDS method). Then protein was cross-linked by glutaraldehyde.

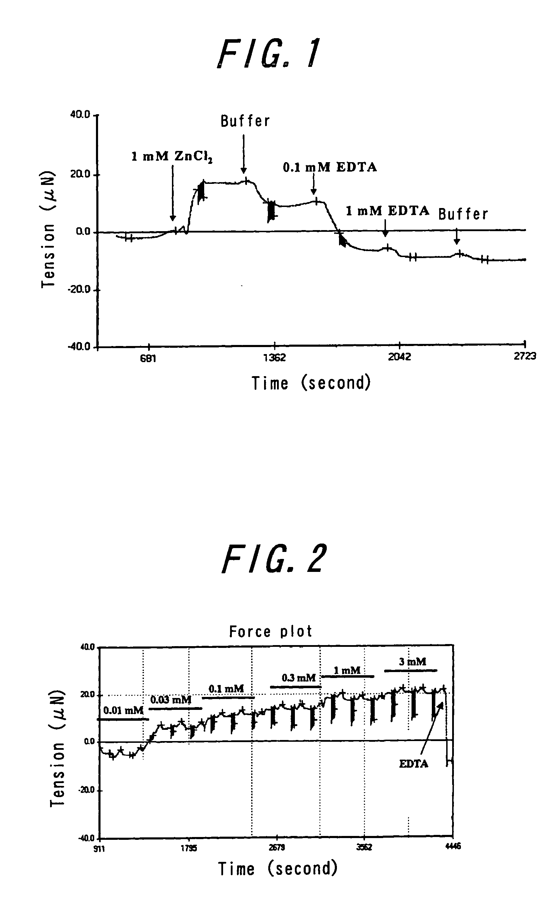

The resulting film was placed on an apparatus having a mechanochemical sensor which was described in WO 2002-503332 or U.S. Patent Publication No.6033913, and it was immersed into 10mM Hepes pH7.4 buffer solution (hereinafter referred to as "the buffer solution") containing 0.15 M NaCl. The buffer solution was passed through the film existing on the detecting apparatus at the flow rate of 0.1 to 0.2 mL/min, in order to stabilize the tension of the sample film. Thereafter ZnCl₂ solution dissolved in said buffer solution was passed through to detect changes in tension and elasticity (FIG.1). The fluctuation in the graph of FIG. 1 indicated that isotropic tension and compliance (the inverse of stiffness) of the amyloid β (1-42) film remarkably increased by ZnCl₂.

On the other hand, the effect of ZnCl₂ was assumed to be reversible, for the tension and elasticity of the film were restored by EDTA, a chelator, to the state prior to addition of ZnCl₂ solution. In FIG.1, a place where the line uprises from the horizontal state (i.e. steady state), means a point where tension is given to the sample film, and compliance is detected where the line oscillates. The result in FIG.1 shows that the present invention enables to detect a particular interaction between amyloid β (1-42) and ZnCl₂ within several minutes. It is assumed that the particular interaction referred herein is aggregation of amyloid β (1-42) caused by Zn²⁺, when considered with many reports such as X. Huang et.al., (J.Biol.Chem., 1997, 272, 26464), C.S.Atwood et.al., (J.Biol.Chem., 1998, 273, 12817), or R.A.Cherny et.al., (Neuron, 2001, 30, 665).

Further, concentration of ZnCl₂ was altered from 0.01 mM to 3 mM, in order to examine the effect of ZnCl₂ concentration (FIG.2). As shown in FIG.2, the result indicated that the change in elasticity with respect to ZnCl₂ solution was concentration-dependent manner.

### EXAMPLE 2: Effect of CuCl₂ on the changes in tension and elasticity of amyloid β

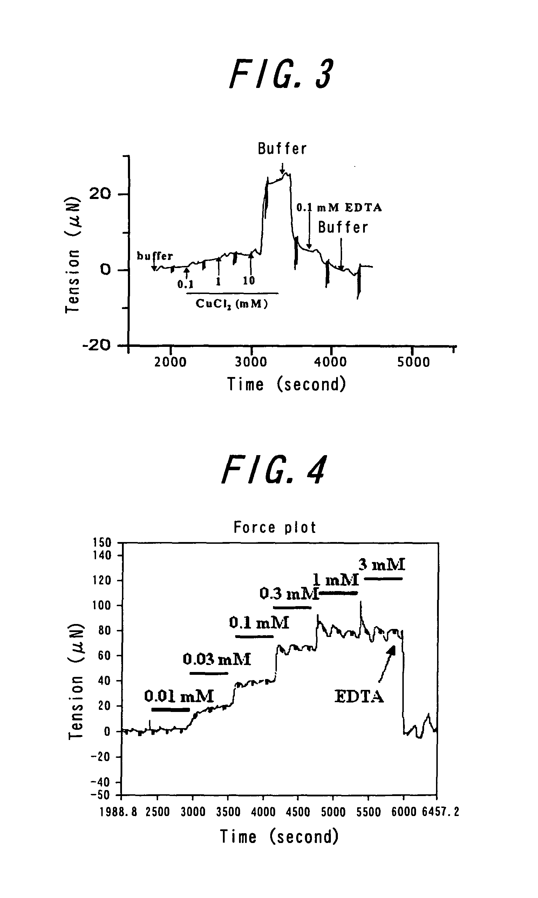

An amyloid β (1-42) membrane was prepared and placed on a detecting apparatus, as described in EXAMPLE 1. The changes in tension and elasticity of the membrane, by CuCl₂ dissolved in the buffer solution, were examined (FIG.3). As same as EXAMPLE 1, concentration-dependent change in tension and elasticity was also recognized in the case of CuCl₂. The tension was restored by EDTA almost to the state prior to addition of CuCl₂. It has been reported that Cu²⁺, as well as Zn²⁺, facilitates aggregation of amyloid β. Such knowledge confirms that the phenomenon detected in the present invention is aggregation of amyloid β.

### EXAMPLE 3: Effect of ZnCl₂ on the changes in tension and elasticity of α-synuclein.

A α-synuclein (BIOMOL International LP, PA, USA) film was prepared and placed on a detecting apparatus, as described in EXAMPLE 1. The changes in tension and elasticity of the film, by ZnCl₂ dissolved in the buffer solution, were examined (FIG.4). As same as EXAMPLE 1, ZnCl₂ concentration-dependent change in tension and elasticity was also recognized in the case of α-synuclein. The tension was restored by EDTA almost to the state prior to addition of ZnCl₂.

### (INDUSTRIAL APPLICABILITY)

The present invention provides a method of detecting an amyloidgenic conformational change of a protein using a mechanochemical sensor. The present invention further provides a method of searching a substance having an activity that affects to an amyloidgenic conformational change using said detecting method, as well as a method of searching a therapeutic or diagnostic agent for amyloid-related diseases. The method of the present invention is assumed to contribute to obtain a novel therapeutic or diagnostic agent for amyloid diseases.

## Claims

1. A method of detecting an amyloidgenic conformational change of a protein, the method comprising:
forming a sample film on a substrate, the sample film comprising a protein that arises an amyloidgenic conformational change, a fragment of said protein, a variant of said protein, said protein added with a tag, or an antibody protein against said protein;
placing said substrate comprising said sample film to a force sensor; and
detecting change(s) in tension and/or elasticity of said sample film when a test sample is subjected to said sample film by said force sensor.

2. The method according to Claim 1, wherein said force sensor is a mechanochemical sensor.

3. The method according to Claim 1, wherein said protein that arises an amyloidgenic conformational change is a protein selected from the group consisting of amyloid β protein, immunoglobulin light chain protein, amyloid A protein, transthyretin protein, lysozyme, BriL protein, cystatin C protein, scrapie protein, β2 microglobulin, apolipoprotein A1, gelsolin, pancreatic islet amyloid protein, fibrinogen, prolactin, insulin, calcitonin, atrial natriuretic peptide, α-synuclein, prion protein, huntingtin protein, superoxide dismutase, α1-antichymotrypsin, and tau protein.

4. The method according to Claim 3, wherein said protein that arises an amyloidgenic conformational change is amyloid β protein.

5. A method of searching a substance having an activity that affects to an amyloidgenic conformational change, the method comprising:
forming a sample film on a substrate, the sample film comprising a protein that arises an amyloidgenic conformational change, a fragment of said protein, a variant of said protein, said protein added with a tag, or an antibody protein against said protein;
placing said substrate comprising said sample film to a force sensor; and
detecting change(s) in tension and/or elasticity of said sample film when a test sample is subjected to said sample film by said force sensor.

6. The method according to Claim 5, wherein said force sensor is a mechanochemical sensor.

7. The method according to Claim 5, wherein said protein that arises an amyloidgenic conformational change is a protein selected from the group consisting of amyloid β protein, immunoglobulin light chain protein, amyloid A protein, transthyretin protein, lysozyme, BriL protein, cystatin C protein, scrapie protein, β2 microglobulin, apolipoprotein A1, gelsolin, pancreatic islet amyloid protein, fibrinogen, prolactin, insulin, calcitonin, atrial natriuretic peptide, α-synuclein, prion protein, huntingtin protein, superoxide dismutase, α1-antichymotrypsin, and tau protein.

8. The method according to Claim 7, wherein said protein that arises an amyloidgenic conformational change is amyloid β protein.

9. A method of searching a therapeutic or diagnostic agent for amyloid-related diseases, the method comprising:
forming a sample film on a substrate, the sample film comprising a protein that arises an amyloidgenic conformational change, a fragment of said protein, a variant of said protein, said protein added with a tag, or an antibody protein against said protein;
placing said substrate comprising said sample film to a force sensor; and
detecting change(s) in tension and/or elasticity of said sample film when a test sample is subjected to said sample film by said force sensor.

10. The method according to Claim 9, wherein said force sensor is a mechanochemical sensor.

11. The method according to Claim 9, wherein said protein that arises an amyloidgenic conformational change is a protein selected from the group consisting of amyloid β protein, immunoglobulin light chain protein, amyloid A protein, transthyretin protein, lysozyme, BriL protein, cystatin C protein, scrapie protein, β2 microglobulin, apolipoprotein A1, gelsolin, pancreatic islet amyloid protein, fibrinogen, prolactin, insulin, calcitonin, atrial natriuretic peptide, α-synuclein, prion protein, huntingtin protein, superoxide dismutase, α1-antichymotrypsin, and tau protein.

12. The method according to Claim 11, wherein said protein that arises an amyloidgenic conformational change is amyloid β protein.

13. A sample film comprising a protein that arises an amyloidgenic conformational change.

14. The sample film according to Claim 13, wherein said protein that arises an amyloidgenic conformational change is amyloid β protein.

15. The sample film according to Claim 14, wherein said sample film is formed by depositing said amyloid β protein using an electrospraying method.
